(19) Europäisches Patentamt
European Patent Office
Office européen des brevets

(11) Veröffentlichungsnummer: **0 173 082**
**B1**

(12) # EUROPÄISCHE PATENTSCHRIFT

(45) Veröffentlichungstag der Patentschrift: **31.08.88**

(51) Int. Cl.⁴: **C 07 D 291/06**

(21) Anmeldenummer: **85109376.5**

(22) Anmeldetag: **25.07.85**

(54) **Verfahren zur Herstellung von 6-Methyl-3,4-dihydro-1,2,3-oxathiazin-4-on-2,2-dioxid und dessen nichttoxischen Salzen.**

(30) Priorität: **07.08.84 DE 3429039**

(43) Veröffentlichungstag der Anmeldung: **05.03.86 Patentblatt 86/10**

(45) Bekanntmachung des Hinweises auf die Patenterteilung: **31.08.88 Patentblatt 88/35**

(84) Benannte Vertragsstaaten: **AT BE CH DE FR GB IT LI NL SE**

(56) Entgegenhaltungen:
**DE-A-2 001 017**
**DE-A-2 434 548**
**DE-A-2 434 549**
**DE-B-2 264 235**
**GB-A-1 476 101**

**ANGEWANDTE CHEMIE, Band 85, Nr. 22, 1973, Seiten 965-973; K. CLAUSS et al.: "Oxathiazinondioxide, eine neue Gruppe von Süssstoffen"**
**CHEMICAL ABSTRACTS, Band 55, Nr. 19, 18. September 1961, Columbus, Ohio, US; C.W. TULLOCK et al.: "Synthesis of fluorides by metathesis with sodium fluoride", Zusammenfassung Nr. 18551g-18552e**
**JOURNAL OF ORGANIC CHEMISTRY, 1960 (25), 2016-2019**

(73) Patentinhaber: **HOECHST AKTIENGESELLSCHAFT, Postfach 80 03 20, D-6230 Frankfurt am Main 80 (DE)**

(72) Erfinder: **Schmidt, Erwin, Dr., Am Flachsland 26, D-6233 Kelkheim (Taunus) (DE)**
Erfinder: **Clauss, Karl, Dr., Im Birkenfeld 20, D-6233 Kelkheim (Taunus) (DE)**

(56) Entgegenhaltungen: (Fortsetzung)
**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 173 082 B1**

LIBER, STOCKHOLM 1988

**Beschreibung**

6-Methyl-3,4-dihydro-1,2,3-oxathiazin-4-on-2,2-dioxid ist die Verbindung der Formel

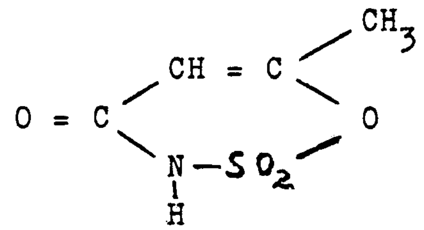

Infolge des aciden Wasserstoffatoms am Stickstoffatom ist die Verbindung zur Salzbildung (mit Basen) befähigt. Die nicht-toxischen Salze - wie z. B. das Na-, das K- und das Ca-Salz - können wegen ihres z. T. intensiven Süßgeschmacks als Süßstoffe auf dem Nahrungsmittelsektor verwendet werden, wobei das K-Salz ("Acesulfam K" oder auch nur "Acesulfam") von besonderer Bedeutung ist.

Zur Herstellung des 6-Methyl-3,4-dihydro-1,2,3-oxathiazin-4-on-2,2-dioxids und dessen nicht-toxischer Salze ist eine Reihe verschiedener Verfahren bekannt; vgl. Angewandte Chemie 85, Heft 22 (1973) S. 965 bis 73, entsprechend International Edition Vol. 12, No. 11 (1973), S. 869-76. Praktisch alle Verfahren gehen von Chlor- oder Fluorsulfonylisocyanat ($XSO_2NCO$ mit X = Cl oder F) aus. Das Chlor- bzw. Fluorsulfonylisocyanat wird dann mit Monomethylacetylen, Aceton, Acetessigsäure, Acetessigsäure-tert.-butylester oder Benzylpropenylether (in einer meist mehrstufigen Reaktion) zu Acetoacetamid-N-sulfochlorid bzw. -fluorid umgesetzt, das unter der Einwirkung von Basen (wie z. B. methanolischer KOH) cyclisiert und die entsprechenden Salze des 6-Methyl-3,4-dihydro-1,2,3-oxathiazin-4-on-2,2-dioxids liefert. Aus den Salzen kann das freie Oxa-thiazinon gewünschtenfalls auf übliche Weise (mit Säuren) erhalten werden.

Ein weiteres Verfahren zur Herstellung der Oxathiazinon-Zwischenstufe Acetoacetamid-N-sulfofluorid geht aus von Amidosulfofluorid $H_2NSO_2F$, dem partiellen Hydrolyseprodukt des Fluorsulfonylisocyanats (DE-OS-2 453 063). Danach wird das Fluorid der Amidosulfonsäure $H_2NSO_2F$ mit einer etwa äquimolaren Menge des Acetoacetylierungsmittels Diketen in einem inerten organischen Lösungsmittel in Gegenwart eines Amins bei Temperaturen zwischen etwa -30 und 100°C umgesetzt; die Umsetzung verläuft nach folgender Reaktionsgleichung (mit Triethylamin als Amin):

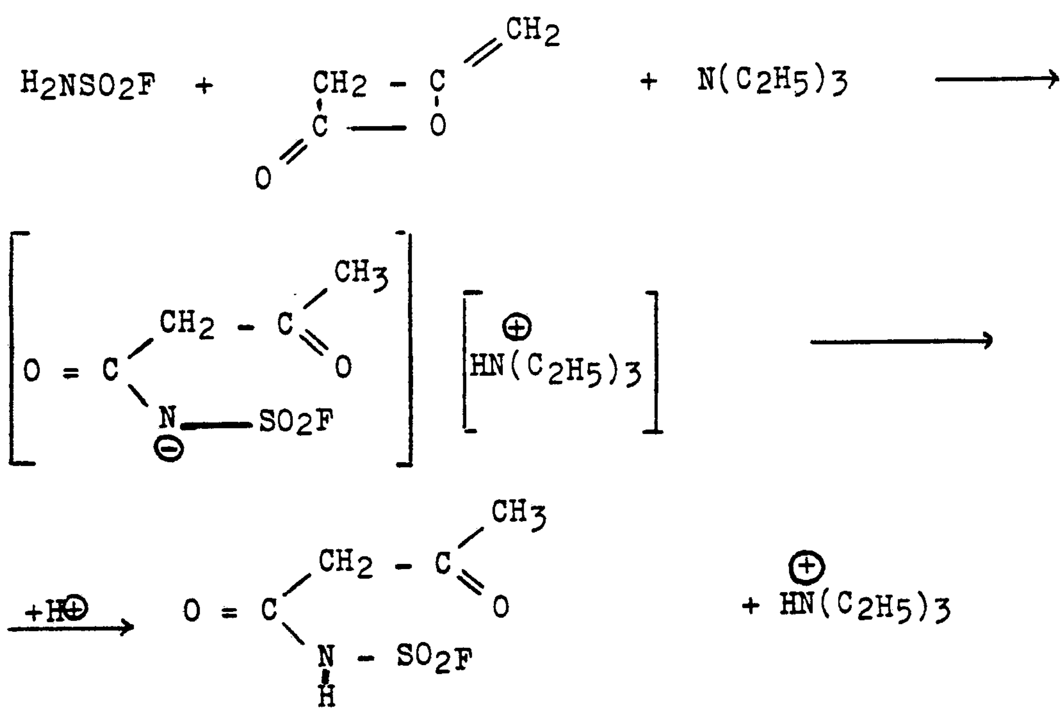

Acetoacetamid-N-sulfofluorid

Das Acetoacetamid-N-sulfofluorid wird dann auf übliche Weise mittels einer Base, z. B. mit methanolischer KOH, zum Süßstoff cyclisiert:

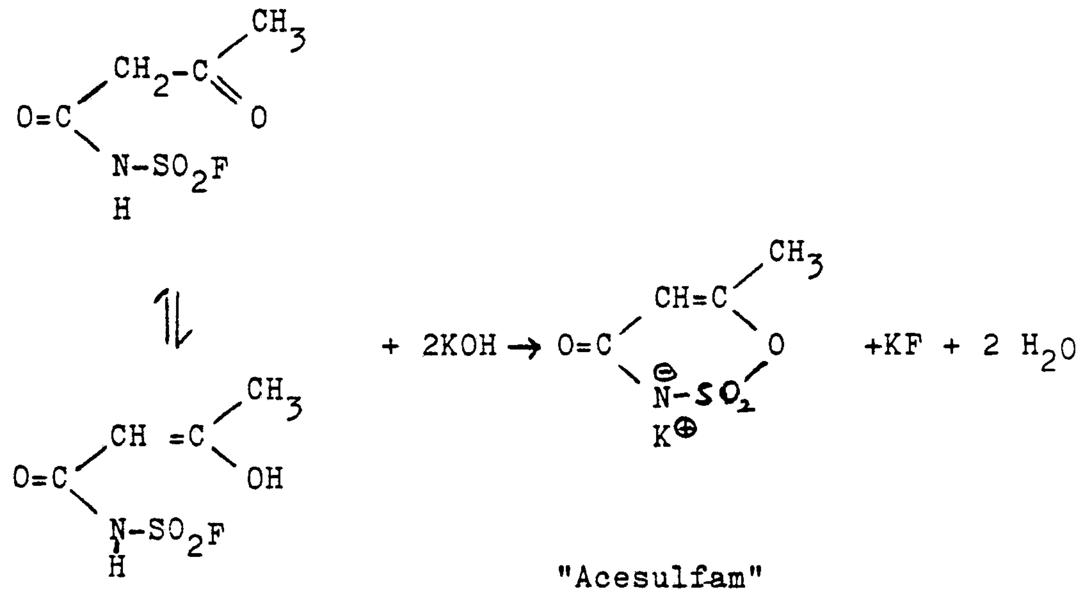


"Acesulfam"

Obwohl die bekannten Verfahren z. T. recht befriedigende Ausbeuten an 6-Methyl-3,4-dihydro-1,2,3-oxathiazin-4-on-2,2-dioxid und dessen nicht-toxischen Salzen liefern, (bis zu ca. 85 % d.Th., bezogen auf das Ausgangs-Amidosulfonsäurehalogenid), sind sie wegen der Notwendigkeit des Einsatzes der nicht ganz einfach zugänglichen Ausgangsstoffe Chlor- bzw. Fluorsulfonylisocyanat vor allem für technische Belange noch verbesserungsbedürftig; die Herstellung des Chlor- und Fluor-sulfonylisocyanats erfordert nämlich wegen der z. T. ziemlich unangenehm handzuhabenden Ausgangsmaterialien - insbesondere von HCN und HF - erhebliche Vorsichtsmaßnahmen und Sicherheitsvorkehrungen. Der Herstellung des Chlor- und Fluor-sulfonylisocyanats liegen folgende Reaktionsgleichungen zugrunde.

$$HCN + Cl_2 \rightarrow ClCN + HCl$$
$$ClCN + SO_3 \rightarrow ClSO_2NCO$$
$$ClSO_2NCO + HF \rightarrow FSO_2NCO + HCl$$

Es wurde daher u. a. bereits vorgeschlagen, 6-Methyl-3,4-dihydro-1,2,3-oxathiazin-4-on-2,2-dioxid und dessen nicht-toxische Salze durch Umsetzung von Acetoacetamid mit der mindestens etwa 2-molaren Menge $SO_3$, gegebenenfalls in einem inerten anorganischen oder organischen Lösungsmittel, mit gegebenenfalls anschließender Neutralisation des dabei in der Säureform gebildeten 6-Methyl-3,4-dihydro-1,2,3-oxathiazin-4-on-2,2-dioxids mit einer Base herzustellen (Patentanmeldung P 34 10 440.2 - HOE 84/F 065).

Bei der Umsetzung entsteht wahrscheinlich zuerst aus einem Mol Acetoacetamid und einem Mol $SO_3$ die Acetoacetamid-N-sulfonsäure, die dann mit einem weiteren Mol $SO_3$ zu dem 6-Methyl-3,4-dihydro-1,2,3-oxathiazin-4-on-2,2-dioxid cyclisiert:

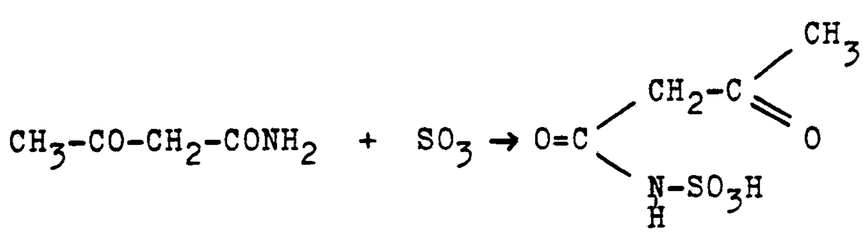

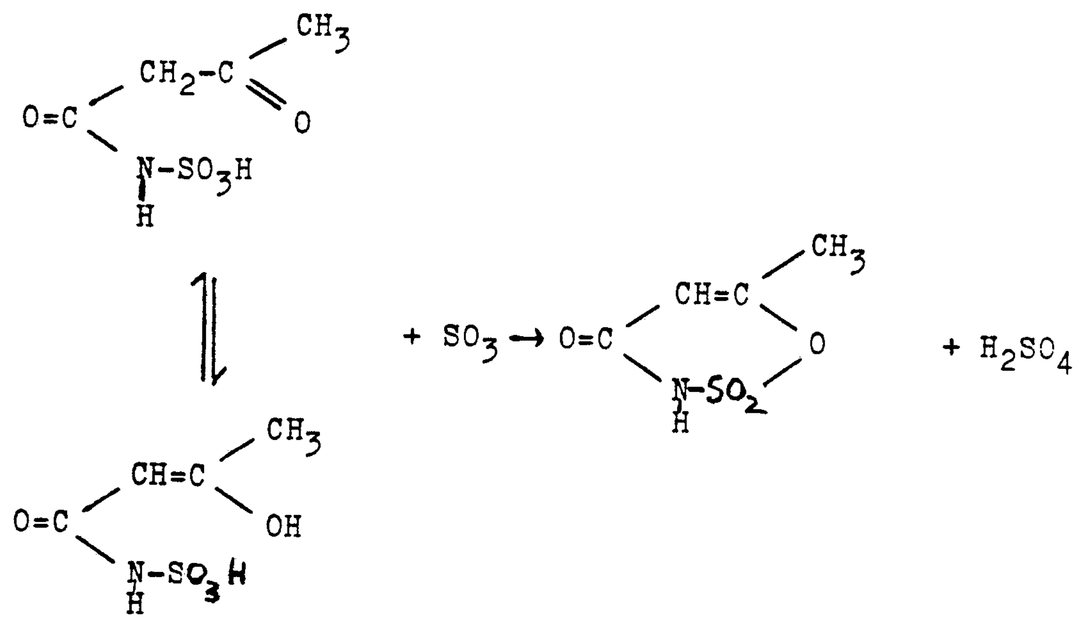


Wenn der Erhalt der Salze beabsichtigt ist, kann das 6-Methyl-3,4-dihydro-1,2,3-oxathiazin-4-on-2,2-dioxid noch - z. B. mit KOH - neutralisiert werden:

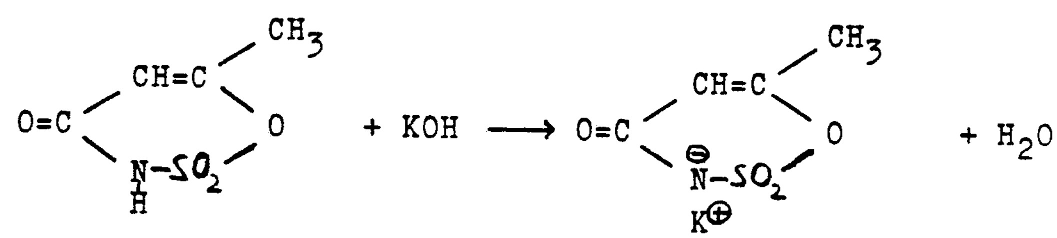


Dabei werden Ausbeuten von etwa 30 bis zu etwa 90 % d.Th., bezogen auf das Acetoacetamid, erhalten.

Wenn man Acetoacetamid anstelle von $SO_3$ mit Sulfurylchlorid $SO_2Cl_2$ umsetzt, erfolgt α-Chlorierung des Acetoacetamids bis zu dem α,α-di-chlorierten Produkt $CH_3$-CO-$CCl_2$-$CONH_2$, welches mit Basen gemäß folgender Reaktionsgleichung gespalten wird:

$CH_3$-CO-$CCl_2$-$CONH_2$ + NaOH → $CH_3COONa$ + $HCCl_2$-$CONH_2$;

vgl. JP-OS 73-39431, ref. in C.A. Vol. 79 (1973), 65827a.

Überraschenderweise wurde nun gefunden, daß Sulfurylfluorid sowie auch einige spezielle andere Fluorsulfonylverbindungen mit Acetoacetamid und Basen in völlig anderer Weise reagieren, nämlich unter Bildung des 6-Methyl-3,4-dihydro-1,2,3-oxathiazin-4-on-2,2-dioxids bzw. dessen entsprechender Salze.

Erfindungsgegenstand ist daher ein Verfahren zur Herstellung von 6-Methyl-3,4-dihydro-1,2,3-oxathiazin-4-on-2,2-dioxid und dessen nicht-toxischen Salzen, ausgehend von Acetoacetamid und einer F-S-O-Verbindung, das dadurch gekennzeichnet ist, daß man Acetoacetamid mit einer F-S-O-Verbindung der Formel I

$FSO_2Y$ (I)

worin Y = F, Cl,
-$OSO_2F$ oder $OSO_2Cl$,
vorzugsweise nur F,

in Gegenwart von Basen umsetzt. Der Umsetzung liegt die folgende Reaktionsgleichung (mit $K_2CO_3$ als Base) zugrunde:

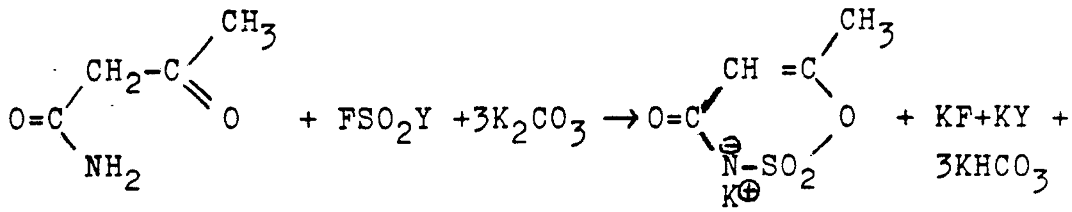

Die nach dem Verfahren erhältlichen Ausbeuten liegen in der Größenordnung der Ausbeute des Verfahrens der vorerwähnten Patentanmeldung und betragen zwischen etwa 20 und 90 % d.Th., bezogen auf das Ausgangs-Acetoacetamid.

Acetoacetamid ist z. B. aus Acetoacetylchlorid oder Diketen und $NH_3$ erhältlich und im übrigen auch ein gängiges Handelsprodukt.

Die unter die Formel I fallenden Verbindungen sind Sulfurylfluorid $SO_2F_2$, Chlorsulfonylfluorid $SO_2ClF$, Pyrosulfurylfluorid $FSO_2$-O-$SO_2F$ und Chlorpyrosulfurylfluorid $ClSO_2$-O-$SO_2F$; bevorzugte Verbindung der Formel I ist Sulfurylfluorid $SO_2F_2$.

Die Herstellung dieser Sulfurylhalogenide erfolgt nach bekannten Verfahren. $SO_2F_2$ und $SO_2ClF$ sind beispielsweise erhältlich durch Erhitzen von $SO_2Cl_2$ mit NaF auf Temperaturen von etwa 60 bis 80°C [C.W. Tullock und D.D. Coffman, J. Org. Chem. 25, S. 2016 (1960)].

Als Basen können für das erfindungsgemäße Verfahren im Prinzip alle möglichen basisch reagierenden Stoffe eingesetzt werden; bevorzugt ist jedoch der Einsatz tertiärer Amine mit insgesamt bis zu 15 C-Atomen, sowie basischer Ionenaustauscher und der Oxide, Hydroxide, Carbonate und Hydrogencarbonate der Alkali- und Erdalkalimetalle.

Beispielhafte tertiäre Amine sind: Trimethylamin, Triethylamin, N-Ethyl-diisopropylamin, Benzyldimethylamin, Dimethylanilin, N,N-Di-methyl-piperazin, N-Ethylpiperidin, Pyridin, α,β- und γ-Picolin, Diaza-bicyclo-octan, Diaza-bicyclo-undecen, etc.

Als basische Ionenaustauscher sind die handelsüblichen Produkte verwendbar.

An Oxiden, Hydroxiden, Carbonaten und Hydrogencarbonaten der Alkali- und Erdalkalimetalle sind in beispielhafter Weise zu nennen:
LiOH, $Li_2CO_3$, $LiHCO_3$,
NaOH, $Na_2CO_3$, $NaHCO_3$,
KOH, $K_2CO_3$, $KHCO_3$,
CaO, $Ca(OH_2)$, $CaCO_3$, $Ca(HCO_3)_2$.

Besonders bevorzugte Basen sind die tertiären Amine mit insgesamt nur bis zu 10 C-Atomen sowie die Hydroxide und Carbonate von Na und K. Ganz besonders bevorzugt ist $K_2CO_3$, weil damit auf besonders einfache Weise das Acesulfam K gewinnbar ist.

Auch Kombinationen mehrerer Basen sind möglich, z. B. zunächst Einsatz eines tert.-Amins mit nachfolgenden Einwirkung von Alkalihydroxid.

Das Acetoacetamid und die F-S-O-Verbindungen der Formel I werden für das erfindungsgemäße Verfahren vorzugsweise im Molverhältnis von etwa 1 : (1 - 1,5) eingesetzt; zum vollständigen Ringschluß werden mindestens etwa 3 - vorzugsweise etwa 3 - 5 Basenäquivalente pro Mol Acetoacetamid eingesetzt. Dabei entsteht das 6-Methyl-3,4-dihydro-1,2,3-oxathiazin-4-on-2,2-dioxid in Salzform, aus der gewünschtenfalls die Säureform auf übliche Weise, z. B. mittels Mineralsäuren (Salzsäure, Schwefelsäure, etc.), saurer Salze ($KHSO_4$ etc.) oder saurer Ionenaustauscher, gewonnen werden kann.

Die erfindungsgemäße Umsetzung kann sowohl in Ab- als auch in Anwesenheit von inerten - d. h. unter den Reaktionsbedingungen mit den Ausgangs- und Endstoffen nicht in unerwünschter Weise reagierenden - Lösungs- und Verdünnungsmitteln durchgeführt werden.

Geeignet sind sowohl protische als auch aprotische organische Lösungsmittel wie
niedere Alkohole (Methanol, Ethanol, i-Propanol, tert.-Butanol etc.),
niedere aliphatische Halogenkohlenwasserstoffe (Ethylenchlorid, 1,2-Dichlorethan, Chloroform, Tetrachlorkohlenstoff, Tetrachlorethylen, etc.),
aromatische Chlorkohlenwasserstoffe (Chlorbenzol, Chlortoluol, etc.),
Ketone (Aceton, Ethyl-methyl-keton, Cyclohexanon, Acetophenon, etc.),
aliphatische Carbonsäureester (Essigsäureethylester, Essigsäurebutylester, Propionsäuremethylester, Malonsäurediethylester, Bernsteinsäuredimethylester, Essigsäuremethoxyethylester, Glykolmonoacetat, Glykoldiacetat, Cyanessigsäureethylester, etc.),
aromatische Carbonsäureester (Benzoesäuremethylester, Benzoesäureethylester, etc.),
aliphatische Carbonsäureamide (Dimethylformamid, Dimethylacetamid, etc.),
Harnstoffderivate (Tetramethylharnstoff, Tetrabutylharnstoff etc.),
aliphatische und aromatische Nitrile (Acetonitril, Benzonitril, etc.).

Die Lösungs- und Verdünnungsmittel können sowohl einzeln als auch in Mischung miteinander (auch im

Bereich von Mischungslücken) verwendet werden.

Auch anorganische Lösungsmittel wie z. B. flüssiges $SO_2$ und gegebenenfalls auch Wasser können eingesetzt werden. Der Einsatz von Wasser ist allerdings dann nicht möglich, wenn als Verbindungen der Formel I Cl-haltige Produkte sowie Pyrosulfurylfluorid verwendet werden, da diese mit Wasser schnell und leicht hydrolysieren. Sulfurylfluorid ist - zumindest bei nicht zu hohen Temperaturen gegen Wasser relativ beständig.

Bevorzugte Lösungsmittel sind Acetonitril und wässriges Aceton, insbesondere wässriges Aceton mit einem Wassergehalt von etwa 1 bis 12 Gew.-%.

Die Menge des Lösungs- oder Verdünnungsmittels ist im Prinzip nicht kritisch und sollte so bemessen sein, daß die Reaktionsmischung gut rührbar ist. Nach obenhin ist die Menge des Lösungs- oder Verdünnungsmittels hauptsächlich durch Wirtschaftlichkeitserwägungen begrenzt; zu verdünnte Lösungen sind nicht mehr vorteilhaft.

Auch die Reaktionstemperatur kann in einem ziemlich weiten Bereich variiert werden. Je nach der Wahl der Basen und der Lösungs- oder Verdünnungsmittel kann man die Reaktion von etwa -70°C bis etwa zum Siedepunkt des Lösungs- oder Verdünnungsmittels durchführen. Bei den tieferen Temperaturen verläuft die Reaktion zunehmend langsamer, bei zu hohen Temperaturen nimmt die Ausbeute ab. Im allgemeinen liegt der gängige Temperaturbereich zwischen etwa -70 und +100°C vorzugsweise zwischen etwa -10 und +60°C.

Als Reaktionsdruck ist im allgemeinen Atmosphärendruck am vorteilhaftesten, doch ist es auch möglich, bei Überdruck zu arbeiten; verminderter Druck kommt weniger in Frage.

Zur Durchführung der erfindungsgemäßen Umsetzung können die Reaktionskomponenten im Prinzip in beliebiger Reihenfolge nacheinander oder gleichzeitig in das Reaktionsgefäß eindosiert werden. Eine vorteilhafte Ausführungsform besteht darin, daß man das Acetoacetamid und die Base(n), gegebenenfalls gelöst in einem inerten Lösungs- und Verdünnungsmittel, vorlegt und die F-S-O-Verbindung der Formel I zudosiert.

Die Aufarbeitung des Reaktionsansatzes erfolgt auf übliche Weise.

Wegen der einfachen Ausgangsprodukte und Durchführbarkeit sowie den z. T. auch recht hohen Ausbeuten ist die Erfindung von nicht unerheblichem wirtschaftlichem Wert.

Die Erfindung wird nun durch die folgenden Beispiele näher erläutert.

**Beispiel 1**

In einem mit Trockeneiskühler versehenen Rührkolben wurde die Lösung von
10,1 g (0,1 Mol) Acetoacetamid und
33,0 g (0,33 Mol) Triethylamin in
100 ml Acetonitril vorgelegt. Dazu wurden dann bei -70°C
10,2 g (0,1 Mol) Sulfurylfluorid gasförmig innerhalb von 30 Minuten eingeleitet.

Es wurde 3 Stunden nachgerührt und das Reaktionsgemisch dabei auf Raumtemperatur erwärmen gelassen. Anschließend wurde das Reaktionsgemisch in 90 ml 4n methanolische KOH eingetropft und abgesaugt. Es wurden
7,2 g (36 % d.Th.) Acesulfam K erhalten, dessen IR-Spektrum mit einem authentischen Material identisch war.

**Beispiel 2**

In einem mit Trockeneiskühler versehenen Rührkolben wie in Beispiel 1 wurden vorgelegt:
10,1 g (0,1 Mol) Acetoacetamid,
50,5 g (0,5 Mol) Triethylamin und
100 ml Acetonitril. Dazu wurden
15,3 g (0,15 Mol) Sulfurylfluorid gasförmig innerhalb von 20 Minuten eingeleitet. Anschließend wurde unter Rühren auf Raumtemperatur erwärmen gelassen. Nach 2-stündigem Rühren wurden
230 ml (0,46 Mol) 2n methanolische KOH zugetropft und abgesaugt. Es wurden
9,7 g (48 % d.Th.) Acesulfam K erhalten.

**Beispiel 3**

In eine Lösung von
20,2 g (0,2 Mol) Acetoacetamid und
23,7 g (0,2 Mol) Chlorsulphonylfluorid in
70 ml flüssigem $SO_2$ wurde bei -10°C eine Mischung mit einem Gesamtvolumen von 50 ml aus
40,4 g (0,4 Mol) Triethylamin und flüssigem

$SO_2$ eingetropft. Es wurde 2 Stunden gerührt und dann das flüssige $SO_2$ abdestilliert, zuletzt im Vakuum. Der Rückstand wurde in 400 ml wässrige NaOH eingetropft, unter Eiskühlung mit konzentrierter Salzsäure angesäuert und mit Essigsäureethylester extrahiert. Nach der Behandlung der Essigesterphase mit Tierkohle und $Na_2SO_4$ wurde der Extrakt im Vakuum eingedampft. Es wurden
15 g (ca. 20 % d.Th.) 6-Methyl-3,4-dihydro-1,2,3-oxathiazin-4-on-2,2-dioxid erhalten.

**Beispiel 4**

150 ml Aceton wurden mit unterschiedlichen Mengen Wasser versetzt. Zu diesen Mischungen wurden jeweils
10,1 g (0,1 Mol) Acetoacetamid und
69 g (0,5 Mol) feingepulvertes trockenes $K_2CO_3$ gegeben.
Danach wurde - bei Raumtemperatur beginnend -
15,3 g (0,15 Mol) Sulfurylfluorid gasförmig eingeleitet.
Dabei stieg die Temperatur des Reaktionsgemisches bis etwa 40°C. Es wurde noch 2 Stunden gerührt und abgesaugt. Der Filterrückstand enthielt Acesulfam K, das dünnschichtchromatographisch (Silicagel, Laufmittel Essigsäureethylester/Eisessig 5 : 1) mit einer Vergleichsprobe identifiziert wurde. Der Filterrückstand wurde in ein Gemisch überschüssiger Salzsäure mit Eis eingetragen und mit Essigsäureethylester extrahiert. Der Essigester-Extrakt wurde über $Na_2SO_4$ getrocknet und im Vakuum eingedampft. Es wurde kristallisiertes 6-Methyl-3,4-dihydro-1,2,3-oxathiazin-4-on-2,2-dioxid erhalten, das mit methanolischer KOH in das Acesulfam K überführt wurde. Die Ergebnisse sind in der nachfolgenden Tabelle zusammengestellt. Beim letzten in der Tabelle aufgeführten Versuch wurde das $K_2CO_3$ als 50 %-ige wässrige Lösung eingesetzt.

**Tabelle**

| Zugesetzte Wassermenge | | Ausbeute Acesulfam K | |
|---|---|---|---|
| (ml) | Gew.-%, bez. auf Aceton | g | % d.Th. |
| 0 | - | 3,75 | 23 |
| 2 | 1,7 | 10,86 | 67 |
| 6 | 5,1 | 12,15 | 75 |
| 8 | 6,7 | 14,10 | 86,5 |
| 10 | 8,4 | 12,20 | 75 |
| 12 | 10,1 | 11,54 | 71 |
| 14 | 11,8 | 8,3 | 51 |
| 69 | 58 | 6,4 | 39 |

**Patentansprüche**

1.Verfahren zur Herstellung von 6-Methyl-3,4-dihydro-1,2,3-oxathiazin-4-on-2,2-dioxid und dessen nicht-toxischen Salzen, ausgehend von Acetoacetamid und einer S-O-Verbindung,
dadurch gekennzeichnet, daß man Acetoacetamid mit einer F-S-O-Verbindung der Formel I

$$FSO_2Y \qquad (I),$$

worin
Y gleich F, Cl, $-OSO_2F$ oder $-OSO_2Cl$, vorzugsweise F ist,

in Gegenwart von einer oder mehreren Basen umsetzt.

2. Verfahren nach Anspruch 1, dadurch gekennzeichnet, daß man eine oder mehrere Basen aus der folgenden Reihe verwendet: Tertiäre Amine mit insgesamt bis zu 15 C-Atomen, basische Ionenaustauscher sowie die Oxide, Hydroxide, Carbonate und Hydrogencarbonate der Alkali- und Erdalkalimetalle.

3. Verfahren nach Anspruch 1 oder 2, dadurch gekennzeichnet, daß man eine oder mehrere Basen aus der folgenden Reihe verwendet: Tertiäre Amine mit insgesamt bis zu 10 C-Atomen sowie die Hydroxide und Carbonate von Na und K.

4. Verfahren nach Anspruch 1, dadurch gekennzeichnet, daß als Base Kaliumcarbonat verwendet wird.

5. Verfahren nach mindestens einem der Ansprüche 1 bis 4, dadurch gekennzeichnet, daß man pro Mol Acetoacetamid 1 - 1,5 Mol F-S-O-Verbindung der Formel I und mindestens 3 Basenäquivalente verwendet.

6. Verfahren nach Anspruch 5, dadurch gekennzeichnet, daß 3 bis 5 Basenäquivalente verwendet werden.

7. Verfahren nach mindestens einem der Ansprüche 1 bis 6, dadurch gekennzeichnet, daß man die Umsetzung in Gegenwart von einem oder mehreren inerten Lösungsmitteln oder Verdünnungsmitteln durchführt.

8. Verfahren nach mindestens einem der Ansprüche 1 bis 7, dadurch gekennzeichnet, daß man die Umsetzung in wäßrigem Aceton, insbesondere in Aceton mit einem Wassergehalt von 1 bis 12 Gew.-%, oder in Acetonitril durchführt.

9. Verfahren nach mindestens einem der Ansprüche 1 bis 8, dadurch gekennzeichnet, daß man die Umsetzung bei einer Temperatur zwischen -70 und +100°C, vorzugsweise zwischen -10 und +60°C durchführt.

10. Verfahren nach mindestens einem der Ansprüche 1 bis 9, dadurch gekennzeichnet, daß man das Acetoacetamid und die Base(n), gegebenenfalls gelöst in einem inerten Lösungs- oder Verdünnungsmittel, vorlegt und die F-S-O-Verbindung der Formel I zudosiert.

**Claims**

1. A process for the preparation of 6-methyl-3,4-dihydro-1,2,3-oxathiazin-4-one 2,2-dioxide and its non-toxic salts, starting from acetoacetamide and an S-O compound, wherein acetoacetamide is reacted with an F-S-O compound of the formula I

$$FSO_2Y \qquad (I)$$

in which Y is F, Cl, $-OSO_2F$ or $-OSO_2Cl$, preferably F, in the presence of one or more bases.

2. The process as claimed in claim 1, wherein one or more bases from the following series are used: tertiary amines having a total of up to 15 carbon atoms, basic ion exchangers, and the oxides, hydroxides, carbonates and bicarbonates of alkali metals and alkaline earth metals.

3. The process as claimed in claim 1 or 2, wherein one or more bases from the following series are used: tertiary amines having a total of up to 10 carbon atoms, and the hydroxides and carbonates of Na and K.

4. The process as claimed in claim 1, wherein the base used is potassium carbonate.

5. The process as claimed in at least one of claims 1 to 4, wherein 1 - 1.5 moles of an F-S-O compound of the formula I and at least 3 equivalents of base are used per mole of acetoacetamide.

6. The process as claimed in claim 5, wherein 3 to 5 equivalents of base are used.

7. The process as claimed in at least one of claims 1 to 6, wherein the reaction is carried out in the presence of one or more inert solvents or diluents.

8. The process as claimed in at least one of claims 1 to 7, wherein the reaction is carried out in aqueous acetone, in particular in acetone having a water content of 1 to 12 % by weight, or in acetonitrile.

9. The process as claimed in at least one of claims 1 to 8, wherein the reaction is carried out at a temperature between -70 and +100°C, preferably between -10 and +60°C.

10. The process as claimed in at least one of claims 1 to 9, wherein the acetoacetamide and the base(s), if appropriate dissolved in an inert solvent or diluent, are initially introduced and the F-S-O compound of the formula I is metered in.

**Revendications**

1. Procédé pour préparer le méthyl-6-dihydro-3,4-oxathiazine-1,2,3-one-4-dioxyde-2,2 et ses sels non-toxiques à partir de l'acétoacétamide et d'un composé à la fois oxygéné et sulfuré, procédé caractérisé en ce qu'on fait réagir l'acétoacétamide avec un composé F-S-O répondant à la formule I:

$$FSO_2Y \qquad (I)$$

dans laquelle Y représente F, Cl, $-OSO_2F$ ou $-OSO_2Cl$, de préférence F, en présence d'une ou plusieurs bases.

2. Procédé selon la revendication 1 caractérisé en ce qu'on utilise une ou plusieurs bases prises parmi des amines tertiaires ayant dans l'ensemble au plus 15 atoms de carbone, des échangeurs d'ions basiques et les oxydes, hydroxydes, carbonates et hydrogénocarbonates des métaux alcalins et des métaux alcalino-terreux.

3. Procédé selon l'une des revendications 1 et 2, caractérisé en ce qu'on utilise une ou plusieurs bases prises parmi des amines tertiaire qui contiennent en géneral au plus 10 atomes de carbone, et les hydroxydes et carbonates de Na et de K.

4. Procédé selon la revendication 1 caractérisé en ce qu'on utilise, comme base, le carbonate de potassium.

5. Procédé selon l'une quelconque des revendications 1 à 4, caractérisé en ce qu'on utilise, par mole d'acétoacétamide, de 1 à 1,5 mole du composé F-S-O de formule I et au moins 3 équivalents de base.

6. Procédé selon la revendication 5 caractérisé en ce qu'on utilise de de 3 à 5 équivalents de base.

7. Procédé selon l'une quelconque des revendications 1 à 6, caractérisé en ce qu'on effectue la réaction en présence d'un ou de plusieurs solvants ou diluants inertes.

8. Procédé selon l'une quelconque des revendications 1 à 7, caractérisé en ce qu'on effectue la réaction dans de l'acétone aqueuse, plus particulièrement dans de l'acétone contenant de 1 à 12 % en poids d'eau, ou dans de l'acétonitrile.

9. Procédé selon l'une quelconque des revendications 1 à 8, caractérisé en ce qu'on effectue la réaction à une température comprise entre -70 et +100°C, de préférence entre -10 et +60°C.

10. Procédé selon l'une quelconque des revendications 1 à 9, caractérisé en ce qu'on commence par mettre dans le récipient réactionnel, l'acétoacétamide et la ou les bases, éventuellement en solution dans un solvant ou diluant inerte, et on introduit progressivement le composé F-S-O de formule I.